# EUROPÄISCHE PATENTANMELDUNG

(11) **EP 4 643 855 A2**
(43) Veröffentlichungstag der Anmeldung: **05.11.2025**
(21) Anmeldenummer: 25185007.9
(22) Anmeldetag: 01.10.2021
(51) Int. Cl.: A61K 31/198

(54) **ZUSAMMENSETZUNG ZUR BEHANDLUNG VON HAAREN UND DER HAUT**

(30) Priorität: 02.10.2020 DE 102020125874
(62) Teilanmeldung aus: 21783014.0
(71) Anmelder: Dr. Kurt Wolff GmbH & Co. KG, 33611 Bielefeld (DE)
(72) Erfinder: SCHULZE ZUR WIESCHE, Erik, 33611 Bielefeld (DE); BECKER, Maike, 33611 Bielefeld (DE); VÖLKER, Jörn Michael, 33611 Bielefeld (DE); KOCH, Nadine, 33611 Bielefeld (DE)
(74) Vertreter: Weickmann & Weickmann PartmbB

(57) **Zusammenfassung**

Die vorliegende Erfindung betrifft eine topische Zusammensetzung, die Dimethylglycin und/oder ein Salz von Dimethylglycin enthält und einen pH-Wert von 3,0 bis 5,9 aufweist. Zudem betrifft die vorliegende Erfindung die (kosmetische und/oder medizinische) Verwendung dieser Zusammensetzung zur Behandlung von Haaren und/oder der Haut.

## Beschreibung

Die vorliegende Erfindung betrifft eine Zusammensetzung, die Dimethylglycin und/oder ein Salz von Dimethylglycin enthält. Zudem betrifft die vorliegende Erfindung die (kosmetische und/oder medizinische) Verwendung dieser Zusammensetzung zur Behandlung von Haaren und/oder der Haut.

Das menschliche Haar hat seine Bedeutung als Schutzfunktion für den Körper mittlerweile zu einem großen Teil verloren. Allerdings hat gesundes Haar weltweit eine große kulturelle Bedeutung für Frauen und Männer und wird häufig als Zeichen von Wohlstand und Gesundheit angesehen. Folglich beeinflusst beispielsweise durch Haarausfall bedingtes schütteres Haar oft die Lebensqualität auf negative Weise. Ein durch Hormone wie Androgene hervorgerufener Haarausfall wird als androgenetische Alopezie (Alopecia androgenetica oder auch AGA) bezeichnet und gilt insbesondere bei Männern aber ebenfalls auch bei Frauen als die häufigste Ursache für Haarausfall. Hierbei kann zwischen männlichen und weiblichen Haarausfallmustern unterschieden werden.

Das Haarwachstum und das Nachwachstum von Haaren hängt von einer ausreichenden Nährstoffversorgung der Haarfollikel ab, welche über das Blut bereitgestellt wird. Eine verringerte Durchblutung (Mikrozirkulation) der Kopfhaut kann somit Haarausfall begünstigen oder gar verursachen. Bei glatzköpfigen Männern ist beispielsweise die Durchblutung der Vertex-Region der Kopfhaut signifikant geringer als bei Männern mit normaler Kopfhautbehaarung. Diese signifikant verringerte Mikrozirkulation kann also eine Erklärung für den Haarausfall sowie das ausbleibende Nachwachsen der Haare (Übergang von Telogen zu Anagen) beispielsweise bei der androgenetischen Alopezie sein. Insgesamt kann Haarausfall also als eines der ersten klinischen Anzeichen für einen verminderten Blutdurchfluss der peripheren Gefäße angesehen werden.

Bis heute gibt es nur wenige zugelassene pharmazeutische Behandlungsmöglichkeiten für Haarausfall, wie androgenetische Alopezie, und die bereits vorhandenen Behandlungsmöglichkeiten sind entweder nicht ausreichend wirksam oder gehen mit unangenehmen Nebenwirkungen einher. Ähnlich unerfreulich sieht es beispielsweise bei Behandlungsmöglichkeiten für den akut einsetzenden entzündlich bedingten kreisrunden Haarausfall, der auch als Alopecia areata oder kurz AA bezeichnet wird, und für durch andere Ursachen bedingten Haarausfall aus.

Neben den Haaren als sogenannte Hautanhangsgebilde ist auch die Funktionsfähigkeit der Haut von großer Bedeutung für ein gesundes Erscheinungsbild. Physiologische Effekte (wie beispielweise Entzündungen) die auf die Haare bzw. Haarwurzeln einwirken, betreffen in ähnlicher Art und Weise auch die Haut. Daher ist auch die Behandlung der Haut sowohl aus medizinischen Gründen, wie eine verbesserte Schutzfunktion und gestärkte Barriereeigenschaft, als auch aus rein optisch-ästhetischen Gründen, wie ein glatteres und schlicht schöneres Hautbild, von großer Bedeutung.

Damit besteht weiterhin ein Bedarf an verbesserten Behandlungsmethoden, insbesondere ein Bedarf an Zusammensetzungen, welche topisch gegen Haarausfall eingesetzt werden können, wobei diese Zusammensetzungen keine oder nur vernachlässigbare Nebenwirkungen zeigen sollen. Insbesondere besteht ein Bedarf an Zusammensetzungen, welche pharmazeutischer Natur sind und keine Nebenwirkungen zeigen oder welche kosmetischer Natur sind.

Ebenso besteht ein Bedarf an verbesserten Hautpflegezusammensetzungen, die sowohl das Hautgefühl als auch das Hautbild verbessern und gleichzeitig die Schutz- und Barrierefunktion der Haut stärken.

Ausgehend hiervon war es die Aufgabe der vorliegenden Erfindung, eine gut verträgliche Zusammensetzung zur Behandlung von Haaren und der Haut, insbesondere zur Prävention und/oder Behandlung von Haarausfall, bereitzustellen. Weiterhin sollte sich diese Zusammensetzung topisch anwenden lassen und die Nachteile der aus dem Stand der Technik bekannten Zusammensetzungen überwinden.

Diese Aufgabe wurde überraschend durch die Zusammensetzung gemäß Anspruch 1 und deren Verwendung gemäß Anspruch 8 gelöst. Bevorzugte Ausführungsformen gehen aus den abhängigen Ansprüchen hervor.

Erfindungsgemäß wird die Aufgabe durch die Bereitstellung einer Zusammensetzung gelöst, welche Dimethylglycin und/oder ein Salz von Dimethylglycin umfasst, wobei die Zusammensetzung einen pH-Wert im Bereich von 3,0 bis 5,9 aufweist.

Es hat sich überraschend gezeigt, dass die erfindungsgemäße Zusammensetzung mit Dimethylglycin und/oder einem Salz von Dimethylglycin eine ausgezeichnete Wirksamkeit in der Behandlung von Haarausfall, insbesondere von erblich bedingtem und altersbedingtem Haarausfall, aufweist, wenn der pH-Wert der Zusammensetzung im Bereich von 3,0 bis 5,9 liegt. Die Zusammensetzung aktiviert die Haut und verbessert die Nährstoff- und Sauerstoffversorgung der Haut und der Haarwurzel deutlich. Zudem ist sie auf der Haut medizinisch und kosmetisch äußerst verträglich.

Dimethylglycin (N,N-Dimethylglycin) kommt in Pflanzen, Tieren und dem Menschen vor, wobei es im Menschen nur in sehr geringen Mengen gebildet wird. Es entsteht bei einer mehrstufigen Biosynthese von Glycin aus Cholin als Zwischenprodukt durch Transaminierung von Betain mit Betain-Homocystein-Methylase.

N,N-Dimethylglycin, auch (Dimethylamino)essigsäure, wird durch die folgende chemische Formel 1 dargestellt:

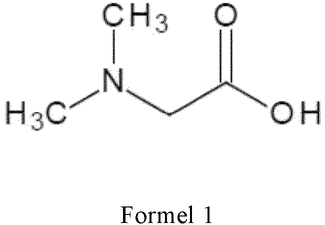

Erfindungsgemäß ist nicht nur der Einsatz von Dimethylglycin, sondern auch der von dessen Salzen, Solvaten und Hydraten. Dabei handelt es sich bevorzugt um pharmazeutisch bzw. kosmetisch annehmbare Salze von Dimethylglycin. Das Salz ist besonders bevorzugt ein wasserlösliches Salz mit einer Löslichkeit in Wasser von mindestens 10 g/l bei 20°C.

In einer bevorzugten Aufführungsform ist das Salz von Dimethylglycin ein Alkali-, Erdalkali- oder Ammoniumsalz von Dimethylglycin.

Beispiele sind Natrium-, Kalium-, Calcium-, Magnesium- und Ammoniumsalze. Bei den Ammoniumsalzen trägt das Ammoniumkation eine bis vier Alkylgruppen mit jeweils unabhängig voneinander 1 bis 4 Kohlenstoffatomen. Bevorzugt sind das Natrium- und Kaliumsalz von Dimethylglycin, insbesondere das Natriumsalz von Dimethylglycin, nämlich Natrium-N,N-dimethylglycinat.

In einer alternativ bevorzugten Ausführungsform kann das Salz von Dimethylglycin das Salz einer anorganischen und/oder organischen Säure mit Dimethylglycin sein.

Beispiele für Salze von Dimethylglycin mit einer anorganischen Säure sind das Hydrochlorid, Hydrobromid, Hydroiodid, Hydrogensulfat, Sulfat, Hydrogensulfit, Sulfit, Hydrogencarbonat, Carbonat, Monophosphat, Diphosphat und Triphosphat von Dimethylglycin sowie Mischungen daraus. Insbesondere bevorzugt ist das Hydrochlorid von Dimethylglycin.

Beispiele für Salze von Dimethylglycin mit einer organischen Säure sind das Acetat, Laktat, Citrat, Succinat, Fumarat, Maleat und Benzoat von Dimethylglycin sowie Mischungen daraus.

Es wird angenommen, dass Dimethylglycin und/oder ein Salz von Dimethylglycin erfindungsgemäß die Zellaktivität und den Sauerstoffumsatz in den Keratinocyten verbessert und damit auch die Zellaktivität in der (Kopf)Haut und in den Haarfollikeln fördert. Ferner wird die Haut geglättet, die Hautbarriere gestärkt und die Wundheilung unterstützt. Es erzielt so erfindungsgemäß eine deutliche haarwurzel- und hautstärkende Wirkung, insbesondere bei der Behandlung von alltags- bzw. altersbedingt gestresster oder geschwächter Haut sowie Haarausfall, wie dem erblich bedingten und altersbedingten Haarausfall.

Der pH-Wert der topischen Zusammensetzung beträgt erfindungsgemäß 3,0 bis 5,9, vorzugsweise 3,5 bis 5,4 und besonders bevorzugt 4,0 bis 5,0 (gemessen bei 21 °C mittels pH-Meter, Mettler-Toledo SevenCompact S220). In diesem Bereich sind die erfindungsgemäßen Zusammensetzungen nicht nur chemisch, physikalisch und mikrobiologisch besonders stabil, sondern auch medizinisch und kosmetisch äußerst verträglich für Haut und Haare. Besonders geeignete pH-Werte für die erfindungsgemäße Zusammensetzung sind: 3,5; 3,6; 3,7; 3,8; 3,9; 4,0; 4,1; 4,2; 4,3; 4,4; 4,5; 4,6; 4,7; 4,8; 4,9; 5,0; 5,1; 5,2; 5,3; und 5,4. Der pH-Wert der Zusammensetzung wird bevorzugt eingestellt mithilfe eines oder mehrerer pH-Modifizierungsmittel. Geeignete pH-Modifizierungsmittel können Säuren, Basen und/oder Puffersysteme sein, um den pH-Wert der Zusammensetzung zu stabilisieren oder zu beeinflussen. Typische pH-Modifizierungsmittel gemäß der vorliegenden Erfindung sind Adipin-, Zitronen-, Äpfel-, Bernstein-, Wein-, Ascorbin-, Phosphor-, Milch- und Fumarsäure sowie die entsprechenden Salze, sowie Natriumalginat, Polyacrylsäure, Natriumcarbonat und Natriumbicarbonat. Im Zusammenhang mit pH-Modifizierungsmitteln bezieht sich der Begriff Salz auf Alkalimetallsalze oder Erdalkalimetallsalze, sofern nichts anderes angegeben ist.

Die erfindungsgemäße Zusammensetzung enthält Dimethylglycin und/oder ein Salz von Dimethylglycin vorzugsweise in einem Anteil von 0,00001 Gew.-% bis 25,0 Gew.-%, bezogen auf das Gesamtgewicht der Zusammensetzung. In einer bevorzugten Ausführungsform enthält die erfindungsgemäße Zusammensetzung Dimethylglycin und/oder ein Salz von Dimethylglycin in einem Anteil von 0,001 Gew.-% bis 10,0 Gew.-%, bevorzugter von 0,01 Gew.-% bis 8,0 Gew.-%, mehr bevorzugt von 0,1 Gew.-% bis 6,0 Gew.-%, noch mehr bevorzugt von 0,3 Gew.-% bis 5,0 Gew.-%, insbesondere von 0,5 Gew.-% bis 3,0 Gew.-%, jeweils bezogen das Gesamtgewicht der Zusammensetzung. In einer bevorzugten Ausführungsform der Erfindung kann die erfindungsgemäße Zusammensetzung 0,1 Gew.%, 0,2 Gew.%, 0,3 Gew.%, 0,4 Gew.%, 0,5 Gew.%, 0,6 Gew.%, 0,7 Gew.%, 0,8 Gew.%, 0,9 Gew.%, 1,0 Gew.%, 1,1 Gew.%, 1,2 Gew.%, 1,3 Gew.%, 1,4 Gew.%, 1,5 Gew.%, 2,0 Gew.% oder 2,5 Gew.% Dimethylglycin und/oder ein Salz von Dimethylglycin enthalten, jeweils bezogen auf das Gesamtgewicht der Zusammensetzung. Bevorzugt enthält die erfindungsgemäße Zusammensetzung Dimethylglycin und/oder ein Salz von Dimethylglycin als chemischen Reinstoff, einschließlich der jeweiligen Solvate und Hydrate (z.B. des Dihydrats von Natriumdimethylglycinat), da so die Reinheit der Zusammensetzung erhöht und das Auftreten von unerwünschten Nebenwirkungen vermindert werden kann. Aus diesem Grund enthält die erfindungsgemäße Zusammensetzung bevorzugt die chemischen Derivate von Dimethylglycin, ausgewählt aus Methylglycin, Trimethylglycin, (2-Hydroxyethyl)-trimethylammonium, und Trimethylhydroxybutyrobetain, in Konzentrationen von weniger als 0,01 Gew.%, bezogen auf das Gesamtgewicht der Zusammensetzung. Besonders bevorzugt sind die erfindungsgemäßen Zusammensetzungen vollständig frei von diesen Derivaten. Bevorzugt ist Dimethylglycin und/oder ein Salz von Dimethylglycin homogen in einer kontinuierlichen oder diskontinuierlichen wässrigen Phase, besonders bevorzugt in einer kontinuierlichen wässrigen Phase, gelöst.

In einer bevorzugten Ausführungsform enthält die erfindungsgemäße Zusammensetzung mindestens einen weiteren Wirkstoff, wobei der mindestens eine weitere Wirkstoff ausgewählt ist aus Koffein, Menthol, Biotin, Zink PCA, Niacinamid, Panthenol, Ectoin, Ubichinon-10, Taurin, Pantolacton, Echinacea, Tocopherylacetat und Kombinationen davon. Die Kombination von Dimethylglycin bzw. den Salzen von Dimethylglycin mit Koffein ist erfindungsgemäß besonders bevorzugt. Bevorzugt umfasst der Wirkstoff bzw. die Wirkstoffkombination nicht die Verbindungen Methylmethionin, Glycero-Phosphocholin, Phosphatidylcholin, N-Acylethanolamin, und/oder N-Acylphosphatidylethanolamin z.B. mit einem C1-C22-Acylrest.

Der IUPAC-Name von Koffein ist 1,3,7-Trimethyl-3,7-dihydro-1H-purin-2,6-dion. Alternativ wird Koffein auch als 1,3,7-Trimethylxanthin bezeichnet. Koffein wird durch die folgende chemische Formel 2 dargestellt.

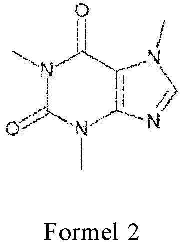

Koffein ist ein zur Klasse der Methylxanthine gehörendes Methylxanthinalkaloid. Es ist eine bittere kristalline Substanz, kann als Purinderivat betrachtet werden und ist chemisch mit den Adenin- und Guaninbasen der Desoxyribonukleinsäuren und der Ribonukleinsäure verwandt.

So zeigt die erfindungsgemäße Wirkstoffkombination von Dimethylglycin bzw. einem Salz von Dimethylglycin mit Koffein eine verbesserte Wirksamkeit in der Behandlung von Haarausfall, insbesondere von erblich bedingtem und altersbedingtem Haarausfall, als Koffein oder Dimethylglycin und/oder ein Salz von Dimethylglycin alleine. Die Wirkstoffkombination steigert dabei die Mikrozirkulation in der Kopfhaut in unerwartetem Maße und verbessert die Nährstoff- und Sauerstoffversorgung der Haarwurzel deutlich.

In einer bevorzugten Ausführungsform enthält die erfindungsgemäße Zusammensetzung Koffein in einem Anteil von 0,001 Gew.-% bis 3,0 Gew.-%, mehr bevorzugt 0,005 Gew.-% bis 2,50 Gew.-%, noch mehr bevorzugt von 0,01 Gew.-% bis 2,0 Gew.-%, insbesondere von 0,1 Gew.-% bis 1,5 Gew.-%, jeweils bezogen auf das Gesamtgewicht der Zusammensetzung. In einer bevorzugten Ausführungsform der Erfindung kann die erfindungsgemäße Zusammensetzung 0,01 Gew.%, 0,05 Gew.%, 0,1 Gew.%, 0,2 Gew.% 0,3 Gew.%, 0,4 Gew.%, 0,5 Gew.%, 0,6 Gew.%, 0,7 Gew.%, 0,8 Gew.%, 0,9 Gew.%, 1,0 Gew.%, 1,1 Gew.%, 1,2 Gew.%, 1,3 Gew.%, 1,4 Gew.%, oder 1,5 Gew.%, Koffein enthalten, jeweils bezogen auf das Gesamtgewicht der Zusammensetzung.

Das Gewichtsverhältnis zwischen Dimethylglycin und/oder einem Salz von Dimethylglycin sowie Koffein liegt vorzugsweise in einem Bereich von 10 : 1 bis 1 : 10, bevorzugt von 6 : 1 bis 1 : 6, mehr bevorzugt von 4 : 1 bis 1 : 4, besonders bevorzugt von 2 : 1 bis 1 : 2. In einer bevorzugten Ausführungsform der Erfindung ist das Gewichtsverhältnis zwischen Dimethylglycin und/oder einem Salz von Dimethylglycin sowie Koffein 0,5; 0,6; 0,7; 0,8; 0,9; 1,0; 1,1; 1,2; 1,3; 1,4; 1,5; 1,6; 1,7; 1,8; 1,9 oder 2,0.

Erfindungsgemäß bevorzugt ist weiterhin die Kombination von Dimethylglycin bzw. einem der Salze von Dimethylglycin, entweder alleine oder besonders bevorzugt zusammen mit Koffein, und einem oder mehreren der folgenden Wirkstoffe.

Alternativ enthält die erfindungsgemäße topische Zusammensetzung gerade kein Koffein. Diese Ausführungsform wird auch als (vollständig) koffeinfreie topische Zusammensetzung bzw. als erfindungsgemäße topische Zusammensetzung ohne Koffein bezeichnet, für die abgesehen davon alle weiteren hierein beschriebenen bevorzugten Merkmale der Erfindung gelten.

Menthol ist ein monocyclischer Monoterpenalkohol und kann der erfindungsgemäßen Zusammensetzung als die Durchblutung stimulierender Wirkstoff zugesetzt werden. Zudem kann Menthol eine erfrischende sensorische Stimulierung der Kopfhaut bewirken.

Biotin, welches auch als Vitamin B₇ oder Vitamin H bezeichnet wird, ist ein wasserlösliches Vitamin aus dem B-Komplex. Biotin kann erfindungsgemäß Haarausfall weiter vermindern und die Haut stärken.

Zink PCA ist das Zinksalz des L-Pyrrolidon-Carboxylats und kann als Substanz mit antimikrobieller Wirkung der erfindungsgemäßen Zusammensetzung zugesetzt werden.

Niacinamid (auch Nicotinamid) ist das Amid der Nicotinsäure und wird auch als Vitamin B₃ bezeichnet. Neben anderen Eigenschaften wie beispielweise einer Verringerung von oxidativem Stress hat das Niacinamid erfindungsgemäß eine das Haarwachstum stimulierende Wirkung.

Panthenol ist ein Provitamin, welches im Körper zu Pantothensäure (Vitamin B₅) umgewandelt wird. Letzteres ist Teil des Coenzyms A und damit für den Hautstoffwechsel wichtig. Bei Einwirkung von Panthenol wird erfindungsgemäß die Hautelastizität und Feuchtigkeit weiter verbessert. Daneben werden Juckreiz und Entzündungen gelindert und die Wundheilung gefördert.

Ectoin ist eine zyklische Aminosäure und liegt in wässriger Lösung als mesomeriestabilisiertes Zwitterion vor. Ectoin wirkt befeuchtend und stabilisiert erfindungsgemäß die natürliche Struktur von Haaren weiter. Zudem hat sich gezeigt, dass Ectoin vor UV-Strahlung schützt und bei der Behandlung entzündlicher Krankheiten hilfreich sein kann.

Ubichinon-10 (Q10 oder Coenzym Q₁₀) ist ein Chinon-Derivat. Das zum Ubichinon-Pool gehörende Q10 gilt als Antioxidans und wirkt erfindungsgemäß stabilisierend auf die Haut und Haare und insbesondere die Haarwurzel.

Taurin oder 2-Aminoethansulfonsäure wirkt erfindungsgemäß als Antioxidans ebenfalls weiter stabilisierend auf die Haut und Haare und insbesondere die Haarwurzel.

Pantolacton entstammt der Gruppe der substituierten Lactone und regt erfindungsgemäß die Wachstumsfaktoren der Haarwurzeln weiter an.

Echinacea wirkt erfindungsgemäß beruhigend auf die Haut und Kopfhaut und lindert auftretenden Juckreiz sowie Spannungen. Zudem kann Echinacea die Blutzirkulation der Kopfhaut anregen und so die Haarfollikel mit sauerstoff- und nährstoffreichem Blut versorgen, was sich weiter stabilisierend auf die Haare und insbesondere die Haarwurzel auswirkt.

Tocopherylacetat zeigt antioxidative Eigenschaften und wirkt erfindungsgemäß weiter stabilisierend auf die Haut und die Haare und insbesondere die Haarwurzel.

In einer bevorzugten Ausführungsform der Erfindung enthält die erfindungsgemäße Zusammensetzung mindestens einen weiteren Wirkstoff ausgewählt aus Menthol, Biotin, Zink PCA, Niacinamid, Panthenol, Ectoin, Ubichinon, Taurin, Pantolacton, Echinacea, Tocopherylacetat und Kombinationen davon jeweils in einem Anteil von 0,001 Gew.-% bis 10,0 Gew.-%, mehr bevorzugt 0,005 Gew.-% bis 7,50 Gew.-%, noch mehr bevorzugt von 0,01 Gew.-% bis 5,0 Gew.-%, insbesondere von 0,1 Gew.-% bis 3,0 Gew.-%, bezogen auf das Gesamtgewicht der Zusammensetzung.

Darüber hinaus kann die erfindungsgemäße Zusammensetzung wasserbasiert sein. Das bedeutet, dass sie beispielsweise 45,0 bis 85,0 Gew.-% Wasser enthält.

Bevorzugt weist die Zusammensetzung eine Viskosität von 800 bis 6000 mPa·s, besonders bevorzugt von 1000 bis 5700 mPa·s und ganz besonders bevorzugt von 2500 bis 5500 mPa·s auf, jeweils gemessen gemäß DIN 53019-1:2008-09 mit dem Rheometer Haake RheoStress1 (ThermoFisher Scientific) bei 20°C und einer Schergeschwindigkeit von 10/s in Platte-Platte Geometrie (Drehkörper PP60 Ti).

In einer Ausführungsform umfasst die Zusammensetzung ein Tensid. Das Tensid kann ein anionisches, nichtionisches, kationisches oder zwitterionisches Tensid sein. Bevorzugt ist das Tensid ein anionisches oder nichtionisches Tensid, insbesondere ein möglichst mildes (d.h. besonders hautverträgliches) anionisches oder nichtionisches Tensid. Nichtionische Tenside werden dabei erfindungsgemäß insbesondere wegen ihrer sehr guten Emulgationseigenschaften sowie ihrer ausgezeichneten Hautpflegeeigenschaften eingesetzt. Anionische Tenside sind bevorzugt, weil sie eine besonders hohe Reinigungsleistung aufweisen. Daher eignen sie sich besonders gut in Reinigungszusammensetzungen wie Shampoos. Kationische Tenside besitzen hervorragende Haarpflegeeigenschaften und werden erfindungsgemäß insbesondere in Haarpflegezusammensetzungen eingesetzt wie in Conditionern, Shampoos und Kuren.

Die erfindungsgemäße Zusammensetzung enthält Tenside vorzugsweise in einer Menge von 2 bis 40 Gew.%, insbesondere von 5 bis 30 Gew.%, bevorzugt von 7 bis 20 Gew.%, besonders bevorzugt von 10 bis 17 Gew.%, jeweils bezogen auf das Gesamtgewicht der Zusammensetzung. Geeignete Mengen an Tensid lauten: 8 Gew.%; 9 Gew.%; 10 Gew.%; 11 Gew.%; 12 Gew.%; 13 Gew.%; 14 Gew.%; 15 Gew.%; 16 Gew.%; 17 Gew.%; 18 Gew.%; 19 Gew.%; 20 Gew.%; 21 Gew.%; 22 Gew.%; 23 Gew.%; 24 Gew.%; 25 Gew.%, jeweils bezogen auf das Gesamtgewicht der Zusammensetzung. Besonders bevorzugt enthält die topische Zusammensetzung der Erfindung ein oder mehrere anionische Tenside in einer Menge von 0,1 bis 20 Gew.%, bevorzugt von 1 bis 17 Gew.% und besonders bevorzugt von 5 bis 15 Gew.%, jeweils bezogen auf das Gesamtgewicht der Zusammensetzung. Geeignete Mengen an anionischem Tensid lauten: 1 Gew.%; 2 Gew.%; 3 Gew.%; 4 Gew.%; 5 Gew.%; 6 Gew.%; 7 Gew.%; 8 Gew.%; 9 Gew.%; 10 Gew.%; 11 Gew.%; 12 Gew.%; 13 Gew.%; 14 Gew.%; 15 Gew.%; 16 Gew.%; 17 Gew.%; 18 Gew.%, 19 Gew.%, 20 Gew.%, jeweils bezogen auf das Gesamtgewicht der Zusammensetzung. In diesen Mengen weisen die Tenside eine besonders hohe Reinigungsleistung auf und sind äußerst verträglich für Haut, Kopfhaut und Haare.

Die Tenside der vorliegenden Erfindung sind u.a. in dem Buch *"*Surfactants and interfacial phenomena", von Milton Rosen und Joy Kunjappu, John Wiley & Sons, Inc.-Verlag, 2012, 4. Auflage beschrieben.

In einer bevorzugten Ausführungsform ist das Tensid ein anionisches Tensid ausgewählt aus Alkylsulfonaten, Alkylsulfaten, Alkylethersulfaten, Alkylphosphaten, Alkylsarkosinaten, Alkyltauraten, Aminosäure-Tensiden und Mischungen davon. Besonders bevorzugt ist das Tensid ausgewählt aus Alkylsulfaten, Alkylsarkosinaten, Alkyltauraten, Alkylglutamat, wie Natriumcocoylglutamat/Dinatriumcocoylglutamat, Alkylgycinat, Alkylalaninat, wie Natriumcocoylalaninat und Mischungen davon. Ebenso bevorzugt wegen ihrer Reinigungsleistung sind Fettalkoholpolyglycerolethersulfate, Monoglyceridsulfate, Mono- und/oder Dialkylsulfosuccinate, Fettsäureisethionate, und α-Olefinsulfonate.

Alkylsulfate haben die generische Formel ROSO₃M, Alkylsarkosinate haben die generische Formel RC(O)N(CH₃)CH₂CO₂M, und Alkyltaurate haben die generische Formel RC(O)N(CH₃)CH₂CH₂SO₃M, wobei R jeweils ein C₄-C₂₆ Alkyl oder C₄-C₂₆ Alkenyl ist und M ein wasserlösliches Kation wie z.B. Ammonium, Natrium oder Kalium darstellt. Vorzugsweise ist M ein Natriumkation. Bevorzugt ist R ein C₁₂-C₁₆ Alkyl oder ein C₁₂-C₁₈ Alkyl.

In einer Ausführungsform ist das Tensid ein nichtionisches Tensid (auch als nichtionischer Emulgator bezeichnet). Nicht-einschränkende Beispiele umfassen Glycerinfettsäureester, Polyoxyethylenether eines oder mehrerer Fettalkohole, alkoxylierte Fettsäurealkylester, Polyglycerinether von Fettalkoholen, Polyglycerinester von Fettsäuren, Polyethylenglykol- und/oder Polypropylenglykolether, Fettsäureamide, Alkylphenolpolyglycolether, Aminoxide und Alkylpolyglucoside.

In einer Ausführungsform ist das Tensid ausgewählt aus der Gruppe der Glycerinfettsäureester, Polyoxyethylenether eines oder mehrerer Fettalkohole, Polyglycerinether von Fettalkoholen, Polyglycerinester von Fettsäuren und deren Mischungen.

In der vorliegenden Erfindung bezieht sich der Begriff "Glycerinfettsäureester" auf einen Glycerinmono- oder Glycerindifettsäureester. Glycerindifettsäureester weisen die Formel R³-COO-(CH₂CH(OH)CH₂)-OOR⁴ oder R³-COO-(CH₂CH(OOR⁴)CH₂)-OH auf. Glycerinmonofettsäureester weisen die Formel R³-COO-(CH₂CH(OH)CH₂)-OH oder HO-(CH₂CH(OOR³)CH₂)-OH auf. Dabei sind R³ und R⁴ unabhängig voneinander aus C₆-C₂₈ Alkyl und C₆-C₂₈ Alkenyl ausgewählt. Glycerinmonofettsäureester enthalten eine Glycerin-Gruppe, welche über eine Esterbindung an eine einzige Fettsäure gebunden. Beispiele sind Glycerinmonostearat, Glycerinmonobehenat, Glycerinmonocaprylat, Glycerinmonocaprat und Glycerinmonolaurat.

Polyoxyethylenether sind Verbindungen der Formel R⁵(OC₂H₃)ₙOH, wobei R⁵ ausgewählt ist aus C₆-C₂₈ Alkyl, C₆-C₂₈ Alkenyl, substituierte und unsubstituierte Phenoxy-Gruppen; und n eine ganze Zahl größer 1 ist. Vorzugsweise wird der Polyoxyethylenether eines oder mehrerer Fettalkohole aus der Gruppe Steareth-2, Steareth-21, Makrogol-Cetostearylether 12, Ceteareth-25, Makrogol-Cetostearylether 20 und Mischungen der vorgenannten Verbindungen ausgewählt. Noch bevorzugter ist der Polyoxyethylenether eine Verbindung ausgewählt aus der Gruppe von Ceteareth-25, Makrogol-Cetostearylether 20 und Mischungen der vorgenannten Verbindungen.

Der Begriff "Polyglycerinether von Fettalkoholen" bezieht sich auf eine Verbindung der Formel R⁶O-(C₃H₆O₂)ₙ-H, wobei R⁶ ein verzweigtes oder lineares C₆-C₂₈ Alkyl oder C₆-C₂₈ Alkenyl ist und n eine ganze Zahl größer als 1, vorzugsweise eine ganze Zahl von 2 bis 10, ist. Es wird bevorzugt, dass die Zusammensetzung 0,01 bis 15,0 Gew.-%, 0,1 bis 10,0 Gew.-% oder 1 bis 5,0 Gew.-% Polyglycerinether enthält.

Der Begriff "Polyglycerinester von Fettsäuren" bezieht sich auf Verbindungen, die sowohl eine Polyglycerineinheit als auch mindestens eine C₆-C₂₆ Alkyl- oder C₆-C₂₆ Alkenylcarbonsäureeinheit enthalten. Diese Verbindungen können die Formel R⁷-R⁸-(C₃H₆O₂)ₙ-H aufweisen, wobei R⁷ ein C₆-C₂₆ Alkanoat- oder C₆-C₂₆ Alkenoat-Rest ist und R⁸ ein geeignetes Verbindungsmolekül oder eine direkte Bindung ist. Somit können die Polyglycerineinheit und die C₆-C₂₆ Alkyl- oder C₆-C₂₆ Alkenylcarbonsäureeinheit direkt durch eine Esterbindung verbunden sein oder eine Verbindungseinheit enthalten, die diese beiden Einheiten miteinander verbindet. Nicht einschränkende Beispiele für diese Gruppe sind Polyglyceryl-3-methylglucosedistearat, Polyglycerinpolycrinoleat, Polyglyceryl-Dimerat-Isostearat, Polyglyceryl-2-Laurat, Polyglyceryl-2-Sesquiisostearat, Polyglyceryl-3-Distearat (Cremophor GS 32), Polyglyceryl-3-Oleat, Polyglyceryl-3-Methylglykose-Distearat, Polyglyceryl-4-Caprat (Polyglycerolcaprat T2010190), Polyglyceryl-4-Diisostearat / Polyhydroxystearat / Sebacat (Isolan GPS) und Polyglyceryl-4-Isostearat.

In einer Ausführungsform umfasst das Tensid ein kationisches Tensid, wie beispielsweise eine quaternäres Tensid. Quaternäre Tenside enthalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkylamidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder - bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

In einer weiteren Ausführungsform enthält die erfindungsgemäße Zusammensetzung mindestens ein Additiv. Bevorzugt sind Additive, die üblicherweise in Shampoos, Conditionern und Emulsionen zur Behandlung der Haut, der Kopfhaut und der Haare eingesetzt werden. Das mindestens eine Additiv kann in einem Anteil von 0,01 Gew.-% bis 12,0 Gew.-%, mehr bevorzugt von 0,25 Gew.-% bis 10,0 Gew.-%, insbesondere von 1,0 bis 7,0 Gew.-% vorliegen.

Das mindestens eine Additiv kann ferner ausgewählt sein aus der Gruppe bestehend aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln und Kombinationen hiervon.

Haarkonditionierungsmittel können statische Aufladungen der Haare durch das Neutralisieren von elektrischer Ladung an ihrer Oberfläche verringern. Beispiele für Haarkonditionierungsmittel sind quartäre Ammoniumverbindungen.

Rückfetter, auch Rückfettungsmittel oder Überfettungsmittel genannt, sind lipophile Substanzen, die eine störende Auswirkung auf die epidermale Barrierefunktion verhindern können. Beispiele für Rückfetter sind Wollwachs, Squalen, flüssiges Paraffin, pflanzliche Öle, Silikone und Cetylpalmitat.

Konservierungsmittel sind Substanzen, die zur Konservierung verwendet werden, indem sie die Zusammensetzung zersetzende Mikroorganismen abtöten und/oder deren Wachstum hemmen. Vorzugsweise können die Konservierungsmittel ausgewählt sein aus der Gruppe bestehend aus Benzoesäure, Benzoesäurederivaten, Sorbinsäure, Sorbinsäurederivaten, Salicylsäure, Salicylsäurederivaten, Phenoxyethanol, Parabenen und Kombinationen hiervon. In einer bevorzugten Ausführungsform werden Natriumbenzoat und/oder Kaliumsorbat als Konservierungsmittel in der erfindungsgemäßen Zusammensetzung eingesetzt. Natriumbenzoat setzt in leicht saurem Milieu Benzoesäure und Kaliumsorbat Sorbinsäure frei. Beiden Säuren wird eine antimikrobielle Wirkung zugeschrieben.

Stabilisatoren können lichtempfindliche Komponenten gegenüber Strahlung schützen und sind bevorzugt UV-Absorber wie beispielsweise Benzophenonderivate.

Die Zugabe von Duftstoffen kann für einen angenehmen Geruch der Zusammensetzung sorgen. Beispiele sind die dem Fachmann bekannten Parfums.

Ein Antioxidans oder Antioxidationsmittel ist eine chemische Verbindung, die eine Oxidation anderer Komponenten in der erfindungsgemäßen Zusammensetzung verlangsamt oder gänzlich verhindert. Als Antioxidantien kommen beispielsweise Zitronensäure, Ascorbinsäure und Butylhydroxyanisol in Frage.

Rheologiemodifikatoren und Verdickungsmittel können dabei helfen, die Applikationseigenschaften der erfindungsgemäßen Zusammensetzung zu verbessern. Als Rheologiemodifikator und Verdickungsmittel kann die Zugabe von Kochsalz (Natriumchlorid) in Betracht gezogen werden. Durch die Zugabe von Kochsalz kann die Fließfähigkeit der erfindungsgemäßen Zusammensetzung in gewissen Grenzen beeinflusst und auf das nötige Maß eingestellt werden. Als Verdicker können zusätzlich natürlich vorkommende Gelbildner eingesetzt werden, die bevorzugt ausgewählt sind aus Agar, Xanthan Gum, Cellulose und oder Cellulose-Derivaten oder Alginsäure.

Im Rahmen der Anmeldung sollen unter Pflegemitteln Substanzen verstanden werden, welche die Haare und/oder die Kopfhaut pflegen. Hydrolysiertes Weizenprotein und Allantoin wirken pflegend auf Kopfhaut und Haare. Hydrolysiertes Weizenprotein hat hauptsächlich feuchtigkeitsspendende Eigenschaften.

Farbstoffe werden optional dazu verwendet, der erfindungsgemäßen Zusammensetzung eine charakteristische Farbe zu verleihen, so dass diese leicht von anderen Produkten unterschieden werden kann. Im Rahmen der Erfindung können Farbstoffe jedoch auch dazu verwendet werden, eine Färbung des menschlichen Haares herbeizuführen.

Als Lösungsmittel kann ein für den Fachmann auf diesem Gebiet gängiges Lösungsmittel oder Lösungsmittelgemisch eingesetzt werden. Bevorzugte Lösungsmittel sind Ethanol oder Butylenglykol, insbesondere 1,4-Butylenglykol, Propylenglykol und Isopropylalkohol. Bevorzugt ist Ethanol. Diese Lösungsmittel können vorzugsweise in einer Menge von 0,1 bis 70 Gew.% in der erfindungsgemäßen Zusammensetzung enthalten sein. Ganz besonders bevorzugt sind sie in einer Menge von 0,1 bis weniger als 5,0 Gew.% enthalten. Lösungsmittel können vorzugsweise in Zusammensetzungen, die bei ihrer Anwendung auf dem Kopf verbleiben (Leave-On-Formulierungen), enthalten sein. Ihre Verwendung kann zu einem Frischegefühl führen und belastet durch das schnelle Abtrocknen das Frisurenbild in einem sehr geringen Maß. Außerdem helfen Lösungsmittel bei der Penetration der Wirkstoffe und verstärken somit deren Wirksamkeit.

Erfindungsgemäß wird die Zusammensetzung topisch angewendet. Unter einer topischen Anwendung ist eine äußerliche Anwendung, insbesondere eine örtliche, äußerliche Anwendung zu verstehen. Erfindungsgemäße Zusammensetzungen sind bevorzugt Haut-, Kopfhaut- und Haarpflegeprodukte (d.h. Behandlungsmittel wie Shampoos, Conditioner, Kuren, Emulsionen, Lotionen, Duschbäder, Tagescreme, Gesichtscreme, Gesichtsfluid und/oder ein Tonikum) und keine (reinen) Styling-Produkte. Mit anderen Worten steht bei den topischen Zusammensetzungen der Erfindung der Zweck des Haar-Stylings, bei dem die Wirkung auf dem Haar selbst und nicht auf der Kopfhaut entfaltet wird, gerade nicht im Vordergrund. Vielmehr liegt der Fokus auf der medizinischen/pharmazeutischen oder kosmetischen Behandlung der Haut, der Kopfhaut und der Haare.

In einer bevorzugten Ausführungsform sind die Bestandteile, insbesondere der Wirkstoff Dimethylglycin bzw. die Salze von Dimethylglycin und die anderen Wirkstoffe zur Behandlung der Haut und der Haare, sofern vorhanden, in der erfindungsgemäßen Zusammensetzung homogen in einer kontinuierlichen oder diskontinuierlichen wässrigen Phase, bevorzugt in einer kontinuierlichen wässrigen Phase, verteilt/gelöst. Der Begriff "wässrige Phase" schließt dabei die mögliche Anwesenheit von mit Wasser mischbaren organischen Lösungsmitteln (z.B. Alkohole) ein. Das bedeutet insbesondere, dass die erfindungsgemäße Zusammensetzung bevorzugt keine weiteren Trägersubstanzen aufweist. Bevorzugt enthält die erfindungsgemäße Zusammensetzung keine lamellare Struktur (insbesondere keine Vesikel und/oder lamellare Doppelmembranstruktur und/oder Liposome). Sie enthält bevorzugt auch keine lamellaren Strukturen (insbesondere Vesikel und/oder lamellare Doppelmembranstrukturen) ausbildende Substanz(en).

In einer bevorzugten Ausführungsform kann die erfindungsgemäße Zusammensetzung in Form einer Leave-On-Formulierung oder Rinse-Off-Formulierung vorliegen.

Eine Leave-On-Formulierung zeichnet sich dadurch aus, dass sie nach der Anwendung in Kontakt mit der zu behandelnden (Kopf)Haut und/oder den zu behandelnden Haaren bleibt. Hierdurch wird ein Art Wirkstoffdepot erzeugt, welches seine Wirksamkeit über einen längeren Zeitraum entfalten kann. Leave-On-Formulierungen können beispielsweise als Hydrogel oder Emulsion formuliert werden oder als wässrige oder wässrig-alkoholische Lösung (Tonikum) vorliegen. Vorzugsweise liegen Leave-On-Formulierungen als leicht viskose Formulierungen vor, sodass sie konzentriert auf der Kopfhaut und/oder den Haaren verbleiben und weniger stark in den Haarschaft spreiten können. Allerdings ist bei diesen Leave-On-Formulierungen zu beachten, dass die Haare durch die Konsistenz gebenden Stoffe nicht belastet werden, da diese dann einen etwas ungepflegten Eindruck hinterlassen.

Eine Rinse-Off-Formulierung zeichnet sich dadurch aus, dass nach der Anwendung die aufgetragenen Komponenten der erfindungsgemäßen Zusammensetzung wieder ausgespült werden. Auf diese Weise kann gegebenenfalls eine zu starke Belastung von Kopfhaut und/oder Haaren vermieden werden. Vorzugsweise können Rinse-Off-Formulierungen in Form eines Shampoos, Conditioners oder einer (Kopf)Haut- und/oder Haarkur vorliegen. In einer bevorzugten Ausführungsform sollte bei einer erfindungsgemäßen Zusammensetzung, die als Rinse-Off Formulierung angewendet wird, eine Einwirkzeit von ca. 2-5 min vorgesehen werden. Auf diese Weise kann die notwendige Penetration der Wirkstoffe in die Kopfhaut und/oder in die Haarwurzeln ermöglicht werden.

Die vorliegende Erfindung bezieht sich weiterhin auf die Verwendung der erfindungsgemäßen Zusammensetzung zur Behandlung von Haaren, der Kopfhaut und/oder der Haut.

In einer bevorzugten Ausführungsform betrifft die Behandlung von Haaren und der Kopfhaut die Behandlung und/oder Vorbeugung von Haarausfall. Dabei umfasst der Begriff Haarausfall Alopecia areata (kreisrunden Haarausfall), Alopecia androgenetica (erblich bedingten Haarausfall) bei Frauen und Männern, diffuse Alopezie (diffusen Haarausfall), altersbedingten Haarausfall (senescent Alopecia) und durch Chemotherapie-bedingten Haarausfall.

Besonders bevorzugt ist der zu behandelnde Haarausfall erblich bedingter Haarausfall (Alopecia androgenetica).

Ebenso besonders bevorzugt ist der zu behandelnde Haarausfall altersbedingter Haarausfall (senescent Alopecia).

In einer bevorzugten Ausführungsform betrifft die Behandlung der Haut die rein optisch-ästhetische Verbesserung, wie die Schaffung eines glatteren und schlicht schöneren Hautbilds. Erfindungsgemäß werden aber auch darüber hinaus gehende kosmetische/medizinische Effekte erzielt, wie eine verbesserte Schutzfunktion und eine gestärkte Barriereeigenschaft. Zudem wird die Wundheilung verbessert.

Anders ausgedrückt führt die erfindungsgemäße Verwendung zu einer deutlichen Verbesserung der epidermalen Barrierefunktionen und der epidermalen Barriereintegrität, zur Verbesserung des Erscheinungsbildes der Haut und der Erhöhung des Feuchtigkeitsgehalts der Haut. Das geht einher mit einer Steigerung der Kohäsion des Stratum corneum und der Homöostase der Hautbarriere und resultiert schließlich in einem verbesserten Schutz vor Infektionen (mikrobiellen Erkrankungen).

Bei medizinischer Verwendung ist erfindungsgemäß sowohl die therapeutische als auch die prophylaktische Behandlung von Hauterkrankungen umfasst. Bei den Erkrankungen handelt es sich bevorzugt um mikrobielle Hautinfektionen, Hautentzündungen, raue Haut, trockene Haut, Hautirritationen, Juckreiz, Pruritus, Allergien, Psoriasis, psoriatische Arthritis, Ekzeme, Scleroderma, atopische Dermatitis, Kontaktdermatitis, systemischen Lupus erythematosus, Akne und Anfälligkeit gegenüber Kontaktallergien.

Die nicht-therapeutische (d.h. rein kosmetische) Verwendung umfasst insbesondere die Behandlung von kosmetischen Indikationen der Haut, insbesondere ausgewählt aus rauer Haut, trockener Haut, Hautirritationen, Juckreiz und Pruritus, sowie die Vorbeugung von Hautinfektionen und die Verminderung der Anfälligkeit gegenüber Kontaktallergien.

Anhand der nachfolgenden Zusammensetzungen soll die Erfindung verdeutlicht werden, ohne sie jedoch auf die speziellen Beispiele einschränken zu wollen.

### Experimenteller Teil

Die folgenden Zusammensetzungen wurden durch für den Fachmann bekannte Homogenisierungsmaßnahmen hergestellt. Die Menge der Komponenten wurde jeweils so gewählt, dass ihr Gewichtsanteil in der fertigen Zusammensetzung angegebenen Gewichtsanteilen entspricht.

### Beispiel 1

Zusammensetzung in Form eines Shampoos (pH 4,8), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Koffein | 1,0 Gew.% |
| Na-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 1a

Zusammensetzung in Form eines Shampoos (pH 4,9), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Na-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 2

Zusammensetzung in Form eines Shampoos (pH 5,3), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Dimethylglycin HCl | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Beispiel 3

Zusammensetzung in Form eines Conditioners (pH 4,4), welches die folgenden Komponenten enthält:

| | |
|---|---|
| Natriumlaureth-11-carboxylat | 1,0 Gew.% |
| Polyquaternium-4/Hydroxypropylstärke Copolymer | 2,0 Gew.% |
| Steareth-8 | 5,0 Gew.% |
| Cetylalkohol | 2,0 Gew.% |
| Parfum | 0,5 Gew.% |
| Na-Dimethylglycinat | 0,4 Gew.% |
| Hydrolysiertes Kreatin | 0,5 Gew.% |
| Zitronensäure | 0,2 Gew.% |
| Natriumbenzoat | 0,05 Gew.% |
| Kaliumsorbat | 0,15 Gew.% |
| Wasser | ad 100 Gew.% |

### Beispiel 4

Zusammensetzung in Form einer Emulsion (pH 5,5), welche die folgenden Komponenten enthält:

| | |
|---|---|
| PEG-40 (hydriertes Rizinusöl) | 6,0 Gew.% |
| Cetearylalkohol | 0,3 Gew.% |
| Cera Alba | 0,7 Gew.% |
| Myristylmyristat | 1,0 Gew.% |
| Hexyldecanol | 5,0 Gew.% |
| Dicaprylether | 4,0 Gew.% |
| Dioctylclyclohexan | 3,0 Gew. % |
| Tocopherylacetat | 1,0 Gew.% |
| Octylmethoxycinnamat | 2,0 Gew.% |
| 4-Isobutyl-dibenzoylmethan | 1,0 Gew.% |
| Ascorbylpalmitat | 0,2 Gew. % |
| Retinylpalmitat | 0,05 Gew.% |
| 1,4-Butylenglykol | 4,0 Gew.% |
| Xanthan Gum | 0,3 Gew. % |
| Hexyldecanol & Hexyldecyllaurat | 1,0 Gew.% |
| Zitronensäure | (pH 5,5) q.s. |
| Na-Dimethylglycinat | 0,4 Gew.% |
| Parfum | 0,2 Gew.% |
| Wasser | ad 100 Gew.% |

### Beispiel 5

Zusammensetzung in Form einer Öl-in-Wasser-Emulsion (pH 4,0), welche die folgenden Komponenten enthält:

| | |
|---|---|
| Glycerinmonostearat | 2 Gew.% |
| Cetylalkohol | 3 Gew.% |
| Paraffinöl, subliquidum | 15 Gew.% |
| Vaseline | 3 Gew.% |
| Caprylcaprinsäuretriglycerid | 4 Gew.% |
| Octyldodecanol | 2 Gew.% |
| hydriertes Kokosfett | 2 Gew.% |
| Glycerin | 3 Gew.% |
| Glykolsäure | (pH 4,0) q.s. |
| Na-Dimethylglycinat | 0,4 Gew.% |
| Parfümöl | q.s. |
| Konservierungsmittel | q.s. |
| Wasser | ad 100 Gew.% |

### Beispiel 6

Zusammensetzung in Form einer Wasser-in-Öl Emulsion (pH 5,9), welche die folgenden Komponenten enthält:

| | |
|---|---|
| PEG-7-hydriertes Ricinusöl | 4 Gew.% |
| Wollwachsalkohol | 1,5 Gew.% |
| Bienenwachs | 3 Gew.% |
| Triglycerid, flüssig | 5 Gew.% |
| Vaseline | 9 Gew.% |
| Stearylalkohol | 4 Gew.% |
| Paraffinöl, subliquidum | 4 Gew.% |
| Glycerin | 2 Gew.% |
| Magnesiumsulfat 7 H2O | 0,7 Gew.% |
| Na-Dimethylglycinat | 0,4 Gew.% |
| Milchsäure | (pH 5,9) q.s. |
| Parfümöl | q.s. |
| Konservierungsmittel | q.s. |
| Wasser | ad 100 Gew.% |

### Beispiel 7

Zusammensetzung in Form eines Shampoos (pH 5,0), welches die folgenden Komponenten enthält:

| | |
|---|---|
| Natriumlaurethsulfat | 12,0 Gew.% |
| Cocoamidopropylbetain | 3,5 Gew.% |
| Disodiumcocoamphodiacetat | 3,0 Gew.% |
| Zitronensäure | 0,2 Gew.% |
| Na-Dimethylglycinat | 1,0 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Referenzbeispiel 1

Zusammensetzung in Form eines Shampoos (ohne Na-Dimethylglycinat; pH 5,1), welches die folgenden Komponenten enthält:

| | |
|---|---|
| Natriummyrethsulfat | 4,0 Gew.% |
| Natriumlaurethsulfat | 4,0 Gew.% |
| Dinatriumlaurethsulfosuccinat | 3,0 Gew.% |
| Tocopherylacetat | 0,3 Gew.% |
| Zitronensäure | 0,2 Gew.% |
| Parfum | 0,3 Gew.% |
| Kaliumsorbat | 0,2 Gew.% |
| Natriumbenzoat | 0,1 Gew.% |
| Wasser | ad 100 Gew.% |

### Referenzbeispiel 2

Zusammensetzung in Form einer Öl-in-Wasser-Emulsion (pH 4,0), welche die folgenden Komponenten enthält:

| | |
|---|---|
| Glycerinmonostearat | 2 Gew.% |
| Cetylalkohol | 3 Gew.% |
| Paraffinöl, subliquidum | 15 Gew.% |
| Vaseline | 3 Gew.% |
| Caprylcaprinsäuretriglycerid | 4 Gew.% |
| Octyldodecanol | 2 Gew.% |
| hydriertes Kokosfett | 2 Gew.% |
| Glycerin | 3 Gew.% |
| Glykolsäure | (pH 4,0) q.s. |
| Parfümöl | q.s. |
| Konservierungsmittel | q.s. |
| Wasser | ad 100 Gew.% |

### Referenzbeispiel 3

Zusammensetzung in Form einer Öl-in-Wasser-Emulsion (pH 8,0), welche die folgenden Komponenten enthält:

| | |
|---|---|
| Glycerinmonostearat | 2 Gew.% |
| Cetylalkohol | 3 Gew.% |
| Paraffinöl, subliquidum | 15 Gew.% |
| Vaseline | 3 Gew.% |
| Caprylcaprinsäuretriglycerid | 4 Gew.% |
| Octyldodecanol | 2 Gew.% |
| hydriertes Kokosfett | 2 Gew.% |
| Glycerin | 3 Gew.% |
| Natriumhydroxid | (pH 8,0) q.s. |
| Na-Dimethylglycinat | 0,4 Gew.% |
| Parfümöl | q.s. |
| Konservierungsmittel | q.s. |
| Wasser | ad 100 Gew.% |

### Studiendesign und -ergebnisse

1. Im Rahmen einer *in vitro* Untersuchung wurde die Wirkung von Na-Dimethylglycinat in einem pH-Bereich von kleiner pH 6 und größer pH 3 im Zellkulturmodell mit humanen hornbildenden Keratinozyten-Zellen untersucht. Hierfür wurden HaCaT-Zellen für 1, 3, 5, und 7 Tage in DMEM-Medium (inklusive fötalem Kälberserum und einem Antibiotika-Antimykotika-Mix) kultiviert und u.a. die Viabilität, Proliferation und Migration der Zellen durch geeignete Messmethoden bestimmt sowie die Expression der für das Zellwachstum relevanten Wachstumsfaktoren bestimmt.
   Nachweis der Viabilität:
      Für diese Messung wurde ein sogenannter MTT-Assay verwendet, um die zelluläre Stoffwechselaktivität als Indikator für die Lebensfähigkeit und Zytotoxizität der Zellen zu bestimmen. Dieser kolorimetrische Assay basiert auf der Reduktion eines gelben Tetrazoliumsalzes (3-(4,5-Dimethylthiazol-2-yl)-2,5-diphenyltetrazoliumbromid oder MTT) zu violetten Formazan-Kristallen durch metabolisch aktive Zellen.
      Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag sowie nach weiteren 24 h sowie 48 h und 72 h Kultivierung bei 37°C und 5 Vol.-% CO₂ wurde das Zellkulturmedium ohne (Kontrolle) bzw. mit den jeweils bereits enthaltenen Wirkstoff-Konzentrationen von Natrium-Dimethylglycinate (DMG) gewechselt. Die Messung erfolgte analog zu bereits publizierten Untersuchungen in B. I. Tóth, N. Dobrosi, A. Dajnoki, G. Czifra, A. Oläh, A. G. Szöllösi, I. Juhäsz, K. Sugawara, R. Paus, T. Bíró, J Invest Dermatol 2011, 131, 1095-1104.

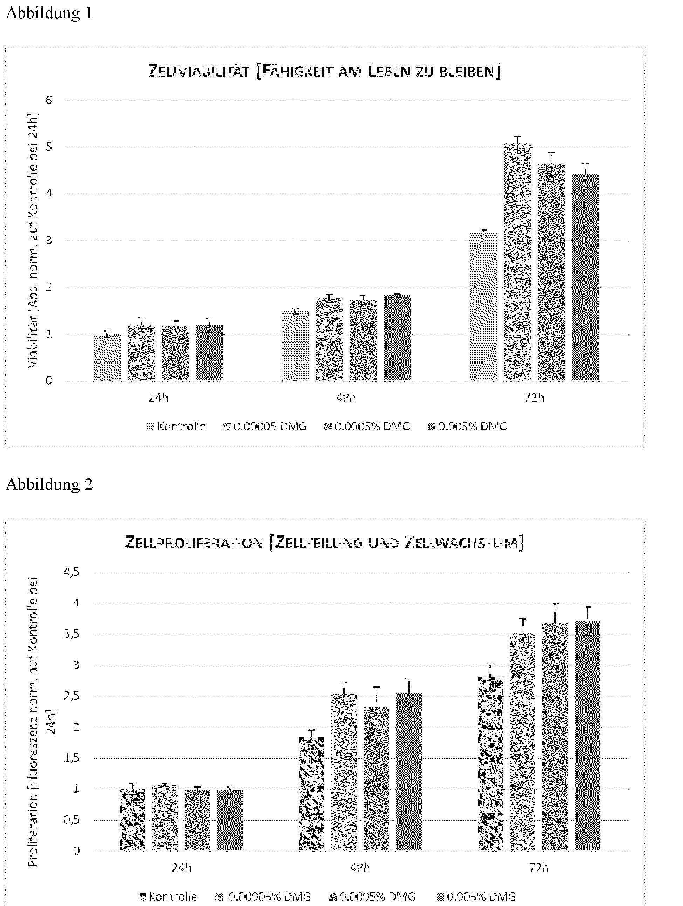

      In Abbildung 1 ist die Zellviabilität nach 24 h, 48 h und 72 h Kultivierung dargestellt und eine deutliche Steigerung durch die Zugabe von DMG nach 48 h und 72 h zu sehen. Die Zellviabilität wurde bestimmt mittels MTT-Assay. Die Absorption wurde jeweils in Vierfachbestimmung gemessen und auf die Kontrolle bei 24 h normiert. Angegeben ist der Mittelwert mit Standardabweichung.
      Nachweis der Proliferation:
      Für die Messung der Proliferation wurde ein sogenannter CyQUANT-Assay durchgeführt. Bei diesem fluoreszenz-basierten Assay bindet der verwendete Fluoreszenzfarbstoff an DNA (engl. deoxyribonucleic acid), wobei der Gehalt an zellulärer DNA ein direktes Maß für die Anzahl an Zellen innerhalb einer Probe ist.
      Humane epidermale Keratinozyten-Zellen (HaCaT) wurden in einer 96-Well Platte mit einer Zelldichte von 5.000 Zellen/Well ausgesät und in Medium (DMEM mit 10% FBS, 1% Penicillin-Streptomycin, 0.5% Fungizone) kultiviert. Am nächsten Tag sowie nach weiteren 24 h sowie 48 h und 72 h Kultivierung bei 37°C und 5 Vol.-% CO₂ wurde das Zellkulturmedium ohne (Kontrolle) bzw. mit den jeweils bereits enthaltenen Wirkstoff-Konzentrationen gewechselt. Die Messung erfolgte analog zu bereits publizierten Untersuchungen in A. Oläh, B. I. Tóth, I. Borbíró, K. Sugawara, A. G. Szöllösi, G. Czifra, B. Päl, L. Ambrus, J. Kloepper, E. Camera, The Journal of clinical investigation 2014, 124, 3713-3724.
      In Abbildung 2 ist die Zellproliferation nach 24 h, 48 h und 72 h Kultivierung dargestellt und eine deutliche Steigerung durch die Zugabe von DMG nach 48 h und 72 h zu sehen. Die Zellproliferation wurde bestimmt mittels CyQUANT-Assay. Die Fluoreszenz wurde jeweils in Dreifachbestimmung gemessen und auf die Kontrolle bei 24 h normiert. Angegeben ist der Mittelwert mit Standardabweichung.
   Nachweis der Migration:
      Für die Messung der Migration wurde ein sogenannter *Wound Healing-*Assay durchgeführt, basierend auf bereits publizierten Untersuchungen in T. Kawabata, T. Otsuka, K. Fujita, G. Sakai, R. Matsushima-Nishiwaki, O. Kozawa, H. Tokuda, International journal of molecular medicine 2018, 42, 3149-3156. Das Prinzip basiert darauf, dass die Migration von Zellen auf eine noch nicht besiedelte Kultivierungsoberfläche im Zeitverlauf gemessen wird.
      Hierfür wurden jeweils 20.000 Zellen in zwei benachbarte und durch einen Silikoneinsatz (mit standardisierter Breite) getrennte Wells bzw. Kavitäten ausgesät und in Medium (DMEM mit 5% FBS, 1% Penicillin-Streptomycin, 0.5% Fungizone) für 48 h bei 37°C und 5 Vol.-% CO₂ kultiviert. Im Anschluss wurde der Plastikeinsatz entfernt ("Erzeugung der Wunde") und die Migration der Zellen durch die Bestimmung der nicht mit Zellen besiedelten Kultivierungsoberfläche im Zeitverlauf durch Bildaufnahmen dokumentiert.
      Parallel dazu wurde das Zellkulturmedium ohne (Kontrolle) bzw. mit den jeweils bereits enthaltenen Wirkstoff-Konzentrationen von DMG direkt nach Entfernung des Plastikeinsatzes (0 h) sowie nach weiteren 24 h gewechselt. Die Auswertung der BIldaufnahmen bzw. Bestimmung der nicht mit Zellen besiedelten Kultivierungsoberfläche erfolgte über eine bestimmte Software (ImageJ).

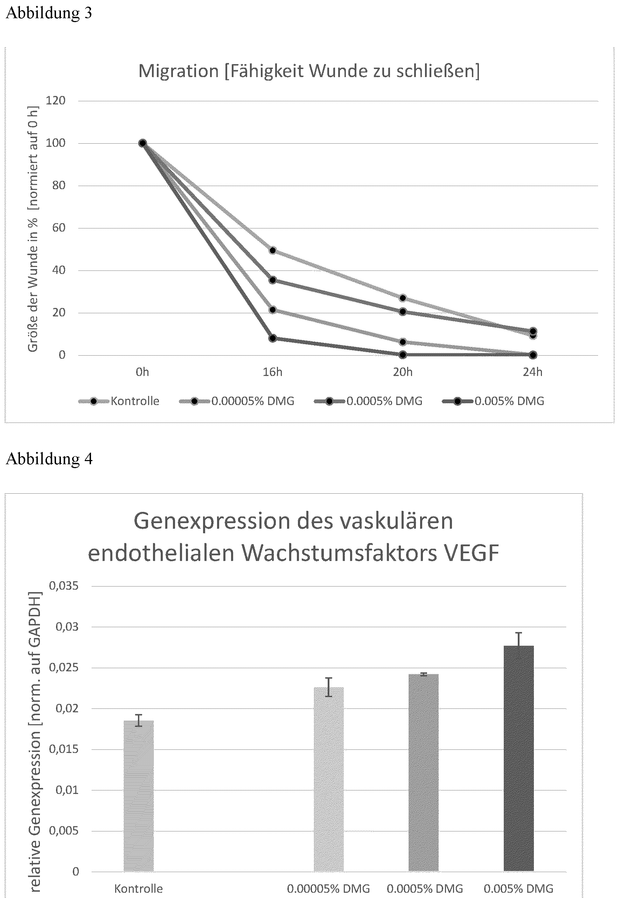

      In Abbildung 3 ist die Migration im Zeitverlauf und unter Einsatz der unterschiedlichen DMG-Konzentrationen dargestellt. Die Migration bzw. Wundschließung erfolgt mit DMG jeweils deutlich schneller im Vergleich zur Kontrolle ohne DMG. Zum Zeitpunkt 0 h wurde die Wunde erzeugt und Bildaufnahmen nach 16 h, 20h bzw. 24 h Kultivierung erstellt. Aus Basis der Bildaufnahmen wurde die Kultivierungsoberfläche, welche noch nicht mit Zellen besiedelt war, mithilfe der ImageJ-Software bestimmt. Die Messwerte sind jeweils auf den Zeitpunkt 0 h (maximale Größe der Wunde) normiert und in % angegeben.
   Nachweis der Genexpression von VEGF:
      VEGF (engl. *Vascular Endothelial Growth Factor*) fördert das Wachstum und die Bildung neuer Blut- und Lymphgefäße. Die Messung der Genexpression erfolgte über ein StandardVerfahren der sogenannten quantitative Echtzeit-PCR (qRT-PCR).
      Humane epidermale Keratinozyten-Zellen (HaCaT) wurden in einer 6-Well Platte mit einer Zelldichte von 140.000 Zellen/Well ausgesät und in Medium (DMEM mit 10% FBS, 1% Penicillin-Streptomycin, 0.5% Fungizone) bei 37°C und 5 Vol.-% CO₂ kultiviert. Am nächsten Tag wurde das Zellkulturmedium ohne (Kontrolle) bzw. mit den jeweils bereits enthaltenen Wirkstoff-Konzentrationen gewechselt und die Zellen nach 24 h geerntet. Die Messung der Genexpression erfolgte mittels qRT-PCR basierend auf bereits publizierten Untersuchungen in B. V. Diaz, M.-C. Lenoir, A. Ladoux, C. Frelin, M. Démarchez, S. Michel, Journal of Biological Chemistry 2000, 275, 642-650.
      In Abbildung 4 ist die Genexpression von VEGF nach 24 h Kultivierung dargestellt und eine deutliche Steigerung der Genexpression durch die Zugabe von DMG zu sehen. Dargestellt ist die mittels qRT-PCR ermittelte relative Genexpression (Dreifachbestimmung, normiert auf die jeweilige Genexpression eines konstitutiv exprimiertes Gens; GAPDH - Glycerinaldehyd-3-phosphat-Dehydrogenase) von VEGF. Angegeben ist der Mittelwert mit Standardabweichung.
      Es hat sich überraschend gezeigt, dass mit DMG im pH Bereich 3,0 bis 5,9 die für das Wachstum relevanten Parameter der HaCaT-Zellen positiv beeinflußt wurden und die Expression des Wachstumsfaktors VEGF signifikant gesteigert wurde gegenüber der Behandlung von HaCaT-Zellen ohne DMG.
2. Das erfindungsgemäße Shampoo aus Beispiel 1 und das Shampoo aus Referenzbeispiel 1 wurden zudem im Rahmen einer Anwendungsstudie bei 30 Männern und 30 Frauen für 6 Monate angewendet. Die Probanden wurden zur subjektiven Bewertung des Rückgangs ihres Haarausfalls nach Beendigung der Anwendung befragt, wobei diese Ergebnisse mit Hilfe von Fragebögen festgehalten wurden. Signifikant weniger Haare pro Tag fielen bei Anwendung des erfindungsgemäßen Shampoos aus Beispiel 1 aus.
3. Die erfindungsgemäße Creme aus Beispiel 5 und die Hautcreme aus dem Referenzbeispiel 2 wurden im Rahmen eines kontrollierten, verblindeten Halbseitentests auf dem Gesicht von 20 Frauen (Phototyp Fitzpatrick I-III**,** Alter: 20-64 Jahre) angewendet. Eine Menge von 80 µL (kontrollierte Volumenabmessung über Gilson Pipetman Microman E, M250E, 50-250 Mikroliter, 7-4305) wurde jeweils zeitgleich auf einer Gesichtshälfte der Probandinnen aufgetragen und gleichmäßig verteilt. Nach einer Minute (Einziehen der Creme in die Haut) wurden Parameter zur Wirksamkeit der Creme subjektiv durch die Bewertung der Probandinnen als auch objektiv durch die Bewertung einer geschulten und lang erfahrenen Kosmetikerin ermittelt. Zu den evaluierten Parametern zählten beispielsweise: Hautgefühl, Ebenmäßigkeit des Hautbildes, Hautfeuchtigkeit.

Es hat sich gezeigt, dass die Probandinnen selbst ein sehr viel gepflegteres und weicheres Hautgefühl erfahren haben durch die Auftragung der erfindungsmäßen Creme im Vergleich zum Referenzbeispiel. Gleichzeitig wurde das Hautbild objektiv als sehr viel gleichmäßiger bewertet nach Anwendung der Testcreme im Vergleich zum Referenzbeispiel.

Die folgenden Punkte sind Gegenstand der Erfindung:
1. Topische Zusammensetzung enthaltend
   Dimethylglycin und/oder ein Salz von Dimethylglycin,
   wobei die Zusammensetzung einen pH-Wert im Bereich von 3,0 bis 5,9 aufweist.
2. Zusammensetzung gemäß Punkt 1, wobei die Zusammensetzung 0,00001 bis 25,0 Gew.% Dimethylglycin und/oder ein Salz von Dimethylglycin enthält.
3. Zusammensetzung gemäß einem der vorangehenden Punkte, wobei die Zusammensetzung Tenside in einer Menge von 2 bis 40 Gew.% enthält.
4. Zusammensetzung gemäß einem der vorangehenden Punkte, wobei die Zusammensetzung eine Viskosität von 800 bis 6000 mPa·s aufweist.
5. Zusammensetzung gemäß einem der vorangehenden Punkte, wobei die Zusammensetzung mindestens einen weiteren Wirkstoff enthält, ausgewählt aus Koffein, Menthol, Biotin, Zink PCA, Niacinamid, Panthenol, Ectoin, Ubichinon-10; Taurin, Pantolacton, Echinacea, Tocopherylacetat und Kombinationen davon.
6. Zusammensetzung gemäß einem der vorangehenden Punkte, wobei die Zusammensetzung mindestens ein Additiv enthält, ausgewählt aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln und Kombinationen hiervon.
7. Zusammensetzung gemäß einem der vorangehenden Punkte, wobei die Zusammensetzung eine Pflegezusammensetzung, bevorzugt zur Förderung des Stoffwechsels der Haut und der Haare, ist.
8. Verwendung der Zusammensetzung gemäß einem der vorangehenden Punkte zur Behandlung von Haaren, der Kopfhaut und/oder der Haut.
9. Verwendung gemäß Punkt 8, wobei die Behandlung der Haare und der Kopfhaut zur Behandlung und/oder Vorbeugung von Haarausfall erfolgt und die Behandlung der Haut zur Verbesserung der epidermalen Barrierefunktionen und der epidermalen Barriereintegrität, zur Verbesserung des Erscheinungsbildes der Haut und zur Erhöhung des Feuchtigkeitsgehalts der Haut sowie zur Verbesserung der Wundheilung erfolgt.
10. Verwendung gemäß Punkt 8 und/oder Punkt 9, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie-bedingtem Haarausfall.
11. Verwendung gemäß einem der Punkte 8 bis 10, wobei die Verwendung eine kosmetische oder medizinische Verwendung ist.

## Patentansprüche

1. Topische Zusammensetzung enthaltend
Dimethylglycin und/oder ein Salz von Dimethylglycin,
wobei die Zusammensetzung einen pH-Wert im Bereich von 3,0 bis 5,9 aufweist.

2. Zusammensetzung gemäß Anspruch 1, wobei die Zusammensetzung 0,00001 bis 25,0 Gew.% Dimethylglycin und/oder ein Salz von Dimethylglycin enthält.

3. Zusammensetzung gemäß einem der vorangehenden Ansprüche, wobei die Zusammensetzung Tenside in einer Menge von 2 bis 40 Gew.% enthält.

4. Zusammensetzung gemäß einem der vorangehenden Ansprüche, wobei die Zusammensetzung eine Viskosität von 800 bis 6000 mPa·s aufweist.

5. Zusammensetzung gemäß einem der vorangehenden Ansprüche, wobei die Zusammensetzung mindestens einen weiteren Wirkstoff enthält, ausgewählt aus Koffein, Menthol, Biotin, Zink PCA, Niacinamid, Panthenol, Ectoin, Ubichinon-10; Taurin, Pantolacton, Echinacea, Tocopherylacetat und Kombinationen davon.

6. Zusammensetzung gemäß einem der vorangehenden Ansprüche, wobei die Zusammensetzung mindestens ein Additiv enthält, ausgewählt aus Haarkonditionierungsmitteln, Rückfettern, Konservierungsmitteln, Stabilisatoren, Duftstoffen, Antioxidantien, Rheologiemodifikatoren, Verdickungsmitteln, Pflegemitteln, Farbstoffen, Perlganzmitteln, Aufhellungsmitteln, Lösungsmitteln und Kombinationen hiervon.

7. Zusammensetzung gemäß einem der vorangehenden Ansprüche, wobei die Zusammensetzung eine Pflegezusammensetzung, bevorzugt zur Förderung des Stoffwechsels der Haut und der Haare, ist.

8. Verwendung der Zusammensetzung gemäß einem der vorangehenden Ansprüche zur Behandlung von Haaren, der Kopfhaut und/oder der Haut.

9. Verwendung gemäß Anspruch 8, wobei die Behandlung der Haare und der Kopfhaut zur Behandlung und/oder Vorbeugung von Haarausfall erfolgt und die Behandlung der Haut zur Verbesserung der epidermalen Barrierefunktionen und der epidermalen Barriereintegrität, zur Verbesserung des Erscheinungsbildes der Haut und zur Erhöhung des Feuchtigkeitsgehalts der Haut sowie zur Verbesserung der Wundheilung erfolgt.

10. Verwendung gemäß Anspruch 8 und/oder Anspruch 9, wobei der Haarausfall ausgewählt ist aus Alopecia areata (kreisrundem Haarausfall), Alopecia androgenetica (erblich bedingter Haarausfall) bei Frauen und Männern, diffuser Alopezie (diffusem Haarausfall), altersbedingtem Haarausfall (senescent Alopecia) und durch Chemotherapie-bedingtem Haarausfall.

11. Verwendung gemäß einem der Ansprüche 8 bis 10, wobei die Verwendung eine kosmetische oder medizinische Verwendung ist.
